Europäisches Patentamt

European Patent Office

Office européen des brevets

(11) Publication number: **0 124 076**
A2

# EUROPEAN PATENT APPLICATION

(21) Application number: **84104652.7**

(22) Date of filing: **25.04.84**

(51) Int. Cl.³: **C 12 N 15/00**, C 12 N 9/28, C 12 N 1/20 // (C12N1/20, C12R1/07),(C12N9/28, C12R1/07)

(30) Priority: **25.04.83 JP 71500/83**

(43) Date of publication of application: **07.11.84**
**Bulletin 84/45**

(84) Designated Contracting States: **DE FR GB**

(71) Applicant: **SANRAKU-OCEAN CO., LTD., 15-1, Kyobashi 1-chome Chuo-ku, Tokyo (JP)**

(72) Inventor: **Aiba, Shuichi, A712, 3-21, Yamada Nishi, Suita-shi Osaka-fu (JP)**
Inventor: **Imanaka, Tadayuki, A205, 3-33, Yamada Nishi, Suita-shi Osaka-fu (JP)**

(74) Representative: **Schön, Alfred, Dr. et al, Patentanwälte Müller-Boré, Deufel, Schön, Hertel Lewald, Otto Isartorplatz 6 Postfach 26 02 47, D-8000 München 26 (DE)**

(54) **Method for preparation of recombinant plasmids, microorganisms transformed by said plasmids and thermo-stable alpha-amylase.**

(57) Disclosed is a method for preparation of recombinant plasmids which contain DNA fragments obtained by the restriction enzyme HindIII from chromosomal DNA's of thermo-stable alpha-amylase-forming thermophilic Bacillus species and can replicate stably in hosts of thermophilic bacteria; microorganisms transformed by said plasmids; and a process for production of thermo-stable alpha-amylase by said microorganisms.

pTB90
6.7 Md

pTB53
11.2 Md

pAT9
11.5 Md

pAT5
16.0 Md

## DETAILED DESCRIPTION OF THE INVENTION

This invention relates to novel recombinant plasmids obtained by genetic engineering techniques and their uses. More particularly, it relates to recombinant plasmids which contain DNA fragments obtained by the restriction enzyme HindIII from chromosomal DNA's of thermo-stable alpha-amylase-forming thermophilic Bacillus species and can replicate stably in hosts of thermophilic bacteria; microorganisms transformed by said plasmids; and a process for production of thermo-stable alpha-amylase by said microorganisms.

The term "thermophilic" used in this specification means that such bacteria can grow and propagate at a temperature over $55^{\circ}$C. Generally speaking, thermophilic bacteria have industrially useful advantages, for example, in operational safety, high growth rate, production of thermo-stable enzymes and reduced cooling cost during cultivation. Thus it has eagerly been hoped that a suitable combination of such hosts and vectors be developed for industrial applications.

In this line of technical approach, the present authors have provided suitable vector plasmids which can be transferred into, and replicate stably in, thermophilic bacteria (Gram-positive or Gram-staining unidentified). (cf. Journal of Bacteriology, Vol. 149, No. 3, pp. 824 - 830, 1982; and Japan Laid-Open Patent Publication No. 57-146799, 1982).

Using the methods described above for preparation of vector plasmids, the present authors have studied to develop recombinant plasmids having practical use. As a result, this invention has been completed by recombining genes of thermo-stable alpha-amylase which are obtained by the restriction enzyme known per se from chromosomal DNA's of thermophilic Bacillus species, with vector plasmids prepared by the above-described methods which can replicate stably in hosts of thermophilic bacteria.

Genes of thermo-stable alpha-amylase according to this invention may be obtained by various restriction enzymes known per se. In practice, however, HindIII is proved to be suitable for effective expression of alpha-amylase activity, as the corresponding DNA fragment has an appropriate molecular weight and include the carrier protein information (signal sequence) that is essential for excretion of protein products from cells. Any vector plasmids may be employed for recombination of said genes of thermo-stable alpha-amylase, irrespective of their origins and molecular weights, as far as they can replicate stably in hosts of thermophilic bacteria and have HindIII restriction sites. Examples of the vector plasmids are those which can replicate stably in thermophilic bacteria belonging to the genus Bacillus, such as pTB19, pTB20 and their derivatives (pTB51, pTB52, pTB53, pTB90, etc.) described in the preceding reference (Journal of Bacteriology, Vol. 149, 824 - 830, 1982). Particularly, pTB53 and pTB90 are the best vector plasmids for this invention, as they can incorporate said genes of thermo-stable alpha-amylase without damage of selection markers of kanamycin resistance ($Km^r$) and tetracycline resistance ($Tc^r$) and have copy numbers of 1 - 9 plasmids per chromosome in hosts of Bacillus species. These vector plasmids were deposited under an accession No. FERM P-5895 with Fermentation Research Institute, Agency of Industrial Science & Technology, Ministry of International Trade and Industry, Japan. Plasmid pTB19 ($Km^r$, $Tc^r$) can be easily prepared from microorganisms carrying pTB19 ($Km^r$, $Tc^r$) by the method described in the above-mentioned reference.

The recombination of genes of thermo-stable alpha-amylase to vector plasmid can be carried out by the so-called shotgun method using restriction enzyme and ligase known per se.

The recombinant plasmids according to this invention are those which contain DNA fragments obtained by the restriction enzyme HindIII from chromosomal DNA's of thermo-stable alpha-amylase-forming thermophilic Bacillus species and can replicate stably in hosts of thermophilic bacteria. More particularly, the recombinant plasmids of this invention contain the DNA fragments obtained by the restriction enzyme HindIII which have a molecular weight of about 4.8 Md and the following restriction sites and products:

(a) 2 BamHI restriction sites; DNA segments of 0.6, 2.1 and 2.1 Md;

(b) 3 EcoRI restriction sites; DNA segments of 0.4, 0.8, 1.3 and 2.3 Md; and

(c) 2 PstI restriction sites; DNA segments of 0.8, 0.8 and 3.2 Md.

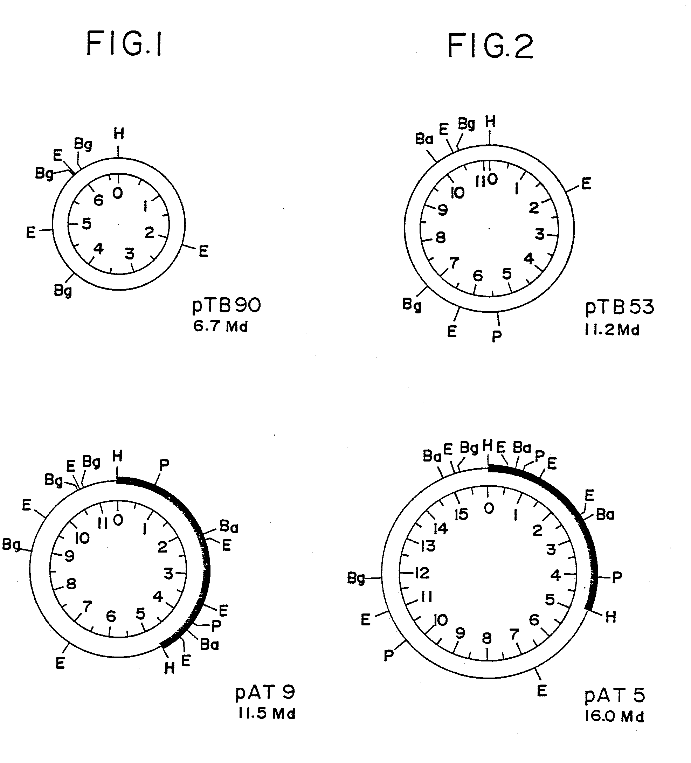

For example, recombinant plasmids designated pAT5 and pAT9 which were produced by the present authors by incorporating the above-described DNA fragments into pTB53 and pTB90, respectively, are suitable, as they possess desirable properties for production of useful materials by genetic engineering techniques such as favorable copy number, type of drug resistance and restriction site. Figs. 1 and 2 show the cleavage maps of these plasmids by various restriction enzymes.

These recombinant plasmids are useful not only for production of alpha-amylase but also for use as vector plasmids for recombination of other DNA fragments, because they include the signal sequence for carrier protein which is essential for production of thermo-stable alpha-amylase as well as for excretion of protein product from cells, as is shown in Figs. 1 and 2; and have the suitable BamHI, PstI and EcoRI restriction sites which are usable in cloning of DNA fragments other than amylase gene.

The second embodiment of this invention relates to novel microorganisms which are obtained by transfer of the above-described recombinant plasmids to thermophilic Bacillus species as hosts. Such microorganisms are very useful for manufacture of thermo-stable alpha-amylase.

One example of such microorganisms is Bacillus stearothermophilus AN174 (pAT9) which was deposited under an accession No. FERM P-7047 with Fermentation Research Institute, Agency of Industrial Science & Technology, April 22nd, 1983, and transferred to the international deposition under the Budapest Treaty and accorded the international deposit number FERM BP-479 on February 3, 1984.

Except for the productivity of thermo-stable alpha-amylase, strain AN174 and its parent, Bacillus stearothermophilus ATCC 12980, show almost identical microbiological properties.

The third embodiment of this invention relates to the manufacture of thermo-stable alpha-amylase by the above-mentioned microorganisms. Media where bacteria belonging to the genus Bacillus can grow are used for production of thermo-stable alpha-amylase according to the present invention. For example, they may contain 4 - 10 % carbon sources such as starch, soluble starch, dextrine and acid hydrolysate of starch; and nitrogen sources such as soybean cake, soybean extract, dry yeast, milk casein, peptone, meat extract, corn steep liquor, peptide-containing material and amino acid may be added singly or in combination in favorable amounts. If required, small amounts of inorganic salts are desirably supplemented. Thermo-stable alpha-amylase is produced by cultivating the above-described microorganisms in such media at a pH of 6.5 - 7.3 and a temperature of 40 - 70°C (preferably 55 - 60°C) for a period of 24 - 72 hours under aerated agitation.

Alpha-amylase thus produced can be recovered from culture broths by a suitable combination of conventional methods. For example, concentration under reduced pressure; precipitation with ammonium sulfate, sodium sulfate, sodium chloride and the like; and solvent fractionation with methanol, acetone and the like are used solely or in suitable combination.

Compared with conventional processes, the method of the present invention has industrial advantages in that the productivity of thermo-stable alpha-amylase is markedly improved, as the gene of thermo-stable alpha-amylase is cloned and amplified in host microorganisms; and that the thermophilic host microorganisms need no cooling during fermentation.

In the following, the present invention will be explained in detail.

Example

(1) Isolation of alpha-amylase-non-producing strain

One loopful of Bacillus stearothermophilus CU21, a thermophilic bacterium (the properties of this strain are described in Imanaka et al.:

Journal of Bacteriology Vol. 149, p. 825, 1982), was inoculated in 5 ml of L broth (tryptone 10 g/l, yeast extract 5 g/l, sodium chloride 5 g/l, pH 7.3) and incubated at $55^{\circ}$C for 16 hours.

A half milliliter of the culture was poured into a 300-ml flask containing 50 ml of LG broth (2.5 g/l of glucose was supplemented to L broth) and incubated at $55^{\circ}$C for about 3 hours. At an optical density of about 0.4 at 660 nm, the cells were collected by centrifugation and resuspended at $48^{\circ}$C in 5 ml of L broth. An N-methyl-N'-nitro-N-nitrosoguanidine solution (N-methyl-N'-nitro-N-nitrosoguanidine was dissolved in Tris-maleate buffer to give a concentration of 1 mg/ml) was added to the cell suspension at a final concentration of 10 µg/ml; and the cell suspension was agitated at $55^{\circ}$C for 15 minutes; diluted in 15 ml of L broth ($48^{\circ}$C) and centrifuged. After washing in about 20 ml of L broth, the cells were resuspended in 20 - 50 ml of L broth and shaken at $55^{\circ}$C for 3 hours. The culture was appropriately diluted, spread on L agar medium and incubated overnight at $55^{\circ}$C. Colonies were transferred on BYS agar medium (Bacto-peptone 10 g/l, meat extract 5 g/l, yeast extract 2 g/l, soluble starch 2 g/l, sodium chloride 2 g/l, agar 15 g/l; pH 7.3) and incubated overnight at $55^{\circ}$C. After an iodine solution (0.01 M $I_2$-KI solution) was poured, colonies producing no haloes were selected. Strain AN174 which formed no alpha-amylase was obtained following 2 runs of the mutagenesis.

(2) Preparation of the chromosomal DNA of strain CU21

Bacillus stearothermophilus CU21 was cultured overnight at $55^{\circ}$C in

a 500-ml flask containing 100 ml of L broth. The cells were recovered by centrifugation and washed in about 20 ml of TE buffer (10 mM Tris buffer, pH 8.5, containing 1 mM ethylenediamine tetraacetate). They were suspended in 6 ml of 15 % sucrose containing 50 mM Tris, pH 8.5, 50 mM EDTA and 1 mg/ml of lysozyme; and allowed to stand for 30 minutes in ice water. The cell suspension was diluted with 6 ml of 2 % sarcosyl containing 5 mM Tris, pH 8.5, and 50 mM EDTA and incubated at room temperature for about 15 minutes until the cells were lysed completely. The lysate (12.0 ml) was added into a 50-ml flask containing 11.0 g of cesium chloride. 0.6 ml of ethidium bromide (whose concentration : 10 mg/ml) was supplemented to the flask. The solution subdivided into two centrifugal tubes were centrifuged with a RP65T rotor (Hitachi Co., Ltd.). Following ultra-centrifugation, the DNA fraction was collected with a syringe and ethidium bromide was completely removed from the DNA fraction by extracting three times with n-butanol. The DNA preparation was dialysed in 10 mM Tris buffer, pH 7.5, containing 0.1 mM EDTA and stored at 4°C.

(3) Treatments with restriction enzyme and ligase

Vector plasmid pTB90 (kanamycin-resistant and tetracycline-resistant; described in Journal of Bacteriology Vol. 149, p. 824, 1982) which can replicate stably in thermophilic bacteria as host was prepared by the conventional method. This pTB90 preparation and the chromosomal DNA of strain CU21 obtained as described above were treated with the restriction enzyme HindIII, respectively. The reaction conditions were as follows:

HindIII buffer (10 mM Tris, pH 7.4, containing 10 mM magnesium chloride, 50 mM sodium chloride and 1 mM dithiothreitol) was used at 10 times strength (10 x HindIII buffer).

| | |
|---|---|
| DNA of strain CU21 | 30 μl |
| 10 x HindIII buffer | 10 μl |
| bovine serum albumin (5 mg/ml) | 2 μl |
| water | 56 μl |
| HindIII solution | 2 μl |
| pTB90 | 20 μl |
| 10 x HindIII buffer | 10 μl |
| bovine serum albumin (5 mg/ml) | 2 μl |
| water | 66 μl |
| HindIII solution | 2 μl |

These reaction mixtures were incubated at 37°C for 1 - 1.5 hours.

The two DNA reaction mixtures were mixed, diluted appropriately with 5 M potassium acetate and then with 0.45 ml of ethanol, and centrifuged. The precipitate was dehydrated and desalted by washing with ethanol and dried in a desiccator. The dry preparation was mixed with 100 μl of 2 x ligase buffer (132 mM Tris, pH 7.6, containing 13.2 mM magnesium chloride), 20 μl of 100 mM dithiothreitol, 20 μl of 5 mM adenosine triphosphate and 60 μl of water. The ligation was started by the addition of 1 μl of DNA ligase solution and the reaction mixture was incubated at 8°C for 16 hours, and, for transformation, diluted with the equivalent volume of 2 x SMM buffer (double-strength buffer containing 0.33 M sucrose, pH 6.5, 0.02 M maleic acid and 0.02 M magnesium chloride).

(4) Preparation of protoplasts

A half milliliter of the overnight culture of Bacillus stearothermophilus AN174 (alpha-amylase-non-producer) at 55°C in L broth was transferred into a 300-ml flask containing 50 ml of LGS broth (LG broth was supplemented with 0.15 M sucrose) and shaken at 55°C for 4 hours. At an optical density of about 0.4 at 660 nm, the cells were collected by centrifugation and suspended in 2 ml of SMM-LG medium (the same volumes of SMM buffer and LG broth were mixed). Lysozyme (50 μg/ml of lysozyme in SMM-LG medium) was added at a final concentration of 1 μg/ml and shaken at 48°C for 20 minutes. After centrifugation, the protoplasts were suspended in 2 ml of SMM-LG medium.

(5) Cloning of the alpha-amylase gene

One and a half milliliter of polyethyleneglycol (40 percent polyethyleneglycol in SMM buffer) was poured into a mixture of 100 μl of the DNA solution from (3) described above and 0.5 ml of the protoplast suspension, and incubated at 48°C for 2 minutes. The mixture was diluted with 5 ml of SMM-LG medium and centrifuged. The pellet was suspended in 1 ml of SMM-LG medium containing 0.01 % bovine serum albumin and gently shaken at 48°C for 1.5 hours.

One tenth milliliter of the protoplast suspension and 1 ml of RGTA containing 25 µg/ml of kanamycin were poured onto an agar plate of RGA containing 25 µg/ml of kanamycin. After the plate was incubated at 48°C for 3 - 5 days, colonies were transferred onto BYS agar medium and incubated at 55°C for one day. Colonies surrounded by haloes contained a recombinant plasmid designated pAT9.

The compositions of RGTA and RGA were as follows:

RGA :   (% in weight/volume)

| | |
|---|---|
| 2.86 % agar + 1.43 % tryptone + 0.71 % yeast extract + 0.71 % NaCl | 700 ml |
| 1 M sucrose | 200 ml |
| 3 % $KH_2PO_4$ + 7 % $K_2HPO_4$ | 50 ml |
| 25 % glucose | 20 ml |
| 1 % casamino acid | 10 ml |
| 2 M magnesium chloride | 10 ml |
| 2 % bovine serum albumin | 10 ml |

RGTA :

The conentration of agar in RGA was reduced to 0.857 %.


(6) Preparation of thermo-stable alpha-amylase

Bacillus stearothermophilus AN174 (pAT9) carrying recombinant plasmid pAT9 was cultivated in L broth at 55°C for 24 hours. The culture was centrifuged and the supernatant solution was dialyzed in 40 mM phosphate buffer, pH 6.0, containing 1 mM calcium chloride and was used as crude enzyme solution.

(7) Measurement of alpha-amylase activity

The activity of alpha-amylase was measured by a conventional method in which soluble starch is employed as substrate and the amount of reducing sugar formed as product is analysed with dinitrosalicylic acid (Bernfeld method; Methods in Enzymology, Vol. 1, pp. 149 - 158, 1955). The following results were obtained:

| Strain | Alpha-amylase activity (units/mg of cells) |
|---|---|
| Bacillus stearothermophilus CU21 | 3.9 |
| Bacillus stearothermophilus AN174 | not detectable |
| Bacillus stearothermophilus AN174(pAT9) | 20.9 |

Provided: One unit of activity is defined as the amount of enzyme per milliliter of enzyme solution which releases reducing sugar equivalent to 1 mg of maltose from soluble starch at 40°C for 3 minutes.

The thermo-stability of this alpha-amylase was confirmed by no loss of activity after heat treatment at 65°C for 60 minutes.

4. BRIEF EXPLANATION OF THE DRAWINGS

Fig. 1 shows the endonuclease restriction maps of vector plasmid pTB90 and its recombinant plasmid pAT9 (the thick solid line of the 4.8 Md HindIII fragment incorporates the thermo-stable alpha-amylase gene).

0124076

Fig. 2 shows the endonuclease restriction maps of vector plasmid pTB53 and its recombinant plasmid pAT5 (the thick solid line has the same meaning as described in Fig. 1).

In the two figures, H, E, P, B and Ba designate cleavage sites with HindIII, EcoRI, PstI, BglII and BamHI, respectively.

Claims

What is claimed is:

1. Recombinant plasmids which contain DNA fragments obtained by the restriction enzyme HindIII from chromosomal DNA's of thermo-stable alpha-amylase-forming thermophilic Bacillus species and can replicate stably in hosts of thermophilic bacteria.

2. The recombinant plasmids according to claim 1 obtained from Bacillus stearothermophilus or Bacillus coagulans as the thermo-stable alpha-amylase-forming thermophilic Bacillus species.

3. The recombinant plasmids according to claim 1 or 2 containing the DNA fragments obtained by the restriction enzyme HindIII which have a molecular weight of about 4.8 megadalton and the following restriction sites:

(a) 2 BamHI restriction sites,

(b) 3 EcoRI restriction sites, and

(c) 2 PstI restriction sites.

4. Thermophilic microorganisms belonging to the genus Bacillus transformed by the recombinant plasmids which contain DNA fragments obtained by the restriction enzyme HindIII from chromosomal DNA's of thermo-stable alpha-amylase-forming thermophilic Bacillus species and can replicate stably in hosts of thermophilic bacteria.

5. The thermophilic microorganism according to claim 4 which is Bacillus stearothermophilus AN174 (pAT9).

6. A process for production of thermo-stable alpha-amylase which is characterized by cultivating in nutrient medium thermophilic microorganisms belonging to the genus Bacillus transformed by the recombinant plasmids which contain DNA fragments obtained by the restriction enzyme HindIII from chromosomal DNA's of thermo-stable alpha-amylase-forming thermophilic Bacillus species and can replicate stably in hosts of thermophilic bacteria whereby thermo-stable alpha-amylase is recovered from culture broth.

0124076

# FIG.1

pTB90
6.7 Md

# FIG.2

pTB53
11.2 Md

pAT 9
11.5 Md

pAT 5
16.0 Md